Europäisches Patentamt

European Patent Office

Office européen des brevets

Publication number: **0 291 173**
**A2**

# EUROPEAN PATENT APPLICATION

Application number: **88303349.0**

Int. Cl.⁴: **A61K 39/012**

Date of filing: **14.04.88**

A request for correction of the second applicant has been filed pursuant to Rule 88 EPC.

Priority: **16.04.87 US 39052**

Date of publication of application:
**17.11.88 Bulletin 88/46**

Designated Contracting States:
**AT BE CH DE ES FR GB GR IT LI LU NL SE**

Applicant: **EMBREX, INC.**
**P.O. Box 13989**
**Research Triangle Park, North Carolina**
**27709-3989(US)**

Applicant: **THE UNITED STATES OF AMERICA**
**as represented by THE SECRETARY OF**
**AGRICULTURE**
**United States Department of Agriculture**
**Washington, DC 20250(US)**

Inventor: **Smith, Harold V.,**
**26 Clover Place**
**Durham North Carolina 27705(US)**
Inventor: **Thaxton, J. Paul,**
**Route 7, Box 82**
**Apex, North Carolina 27502(US)**
Inventor: **Ruff, Michael D.,**
**7104 Westwind Drive**
**Bowie Maryland 20715(US)**

Representative: **Bankes, Stephen C. D. et al**
**Baron & Warren 18 South End Kensington**
**London W8 5BU(GB)**

Method of treating a bird's egg with an immunogen and eggs treated thereby.

Birds are protected against an immunizable disease by administering a nonreplicating immunogen effective for inducing immunity against the disease to the eggs prior to hatching. The method is useful, for example, for immunizing girds against avian coccidiosis with a sporulated Eimeria tenella oocyst extract.

EP 0 291 173 A2

## METHOD OF TREATING A BIRD'S EGG WITH AN IMMUNOGEN AND EGGS TREATED THEREBY.

Avian coccidiosis is a devastating poultry disease largely caused by a variety of different protozoan species in the genus Eimeria. In chickens, the increased cost in broiler production attributable to coccidiosis has been estimated at one-half to one cent per pound. Based on an annual production of 4,500,000,000 broilers annually in the United States, losses would total between 100 and 200 million dollars. To this figure, the annual cost of anticoccidials, estimated at 90 million dollars in the United States, should also be added. These great costs, along with the increasing demand for poultry, emphasize the importance of finding new ways to reduce the incidence of coccidiosis in chickens.

Coccidiosis has a complicated life cycle which consists of both asexual and sexual stages. The cycle is initiated when birds ingest sporulated oocysts (generally associated with fecal material). These oocysts contain the invasive asexual sporozoites which are released into the bird's digestive tract. The sporozoites then invade epithelial cells, and develop into multinucleate structures called schizonts. Each schizont matures and releases numerous invasive asexual structures, known as merozoites, into the bird's digestive tract, where they in turn invade other epithelial cells. These multiple invasive asexual stages, involving both sporozoites and merozoites, produce the pathological digestive tract lesions characteristic of coccidiosis. The sexual stage of the coccidiosis life cycle is initiated when merozoites differentiate into gametocytes. Gametocytes then fuse and the fertilization products, called oocysts, are released in the feces. The release of oocysts completes the parasite's life cycle.

Various species of Eimeria infect a wide range of hosts, including mammals, but nine Eimeria species have been recognized as being pathogenic to varying degrees in birds. These species are Eimeria acervulina, E. mivati, E. mitis, E. praecox, E. hagani, E. necatrix, E. maxima, E. brunetti and E. tenella. In chickens, the life cycle of Eimeria tenella, a representative species, is completed in about seven to nine days.

U.S. Patent No. 4,458,630 to Sharma and Burmester teaches that birds can be immunized against Marek's disease by injecting eggs, prior to hatching, with a replicating viral vaccine. An important advantage of this pioneering procedure is that it provides a way to impart resistance to Marek's disease, and numerous other diseases, in chicks at a very young age. The mechanism by which the Sharma method provides immunity involves deposition of the vaccine into amniotic fluid within the egg, and subsequent infection of the embryo via the embryo's respiratory tract (probably through the inhalation of amniotic fluid containing the vaccine virus). The vaccine virus then replicates to a high titer in the lung of the treated embryo, and spreads to other parts of the embryo's body. See, e.g., J. Sharma, Avian Diseases 29, 1155, 1167-68 (1985).

In view of the mechanism by which the Sharma method is believed to -provide immunity, it has not previously been expected that the administration of a nonreplicating immunogen to a bird prior to hatching would similarly immunize the bird against disease. See also Immunology: Basic Processes, page 14 (J. Bellanti 2d Ed. 1985) (observing that data obtained with nonreplicating antigens may not be applicable to phenomena involving self-replicating immunogens). Nevertheless, some of the most promising approaches for immunization against avian diseases, particularly avian coccidiosis, involve the use of nonreplicating immunogens instead of replicating immunogens. For example, Murray, P.K. et al., Research in Avian Coccidiosis, 564, (McDougald, L.R., Joyner, L.P., and Long, P.L., eds. 1986) (Published by University of Georgia Department of Poultry Science), report that broilers can be vaccinated against coccidiosis shortly after hatching with a nonreplicating immunogen.

The present invention, discussed in detail below, arose from the inventors proceeding against the weight of conventional wisdom and attempting to immunize birds prior to hatching with a nonreplicating immunogen: an attempt which proved successful. These results indicate that the prenatal avian immune system has a memory for immunogens which was not previously known to exist.

Detailed Description of the Invention

Applicants herein disclose a method for controlling an immunizable disease in a bird. The method comprises administering a nonreplicating immunogen effective for inducing immunity against the disease to the bird, wherein the immunogen is administered to the bird while the bird is an embryo enclosed within an egg. The immunogen is administered any time prior to hatching, and is preferably administered during the final quarter of the bird's incubation period prior to hatching. The present invention is particularly useful in immunizing birds against avian coccidiosis, for which an Eimeria extract. preferably a sporulated Eimeria oocyst extract. is used (various exemplary species of Eimeria are listed in the background section above). The invention is particularly useful for the treatment of chickens, but is useful for the treatment of birds in general, including chickens, turkeys, ducks, geese, quail, and pheasants.

Nonreplicating immunogens used in practicing the present invention can be administered by several means, including direct absorption through the eggshell in a suitable carrier such as Dimethylsulfoxide, or by penetration of the shell by injection. Injection is the preferred means of administration, and is discussed in greater detail below.

The process of the present invention is used to produce treated eggs which will provide immunized birds. Accordingly. another aspect of the present invention is the manufacture of a fertile bird egg having deposited therein a foreign, nonreplicating immunogen effective for inducing immunity to avian coccidiosis in the hatchling of the egg. Preferably, these eggs are chicken eggs, which are preferably in the last quarter of their incubation period.

As noted previously, avian coccidiosis is among the most destructive diseases in the poultry industry, and it is this disease to which the present invention is most specifically addressed. Those' skilled in the art will, however, appreciate that the present invention provides an important new procedure for the prevention of other diseases significant in the poultry industry, and therefore extends to all immunizable avian diseases, whether of viral, bacterial, or other microbial origin. Representative examples of such diseases are avian leukosis, reticuloendotheliosis, infectious bronchitis, infectious bursal disease, Newcastle's disease, adenovirus diseases, reovirus, pox, laryngotracheitis, influenza, infectious coryza, fowl typhoid, cryptosporidiosis, and fowl cholera.

The vaccines encompassed by the present invention are limited to nonreplicating immunogens. The term nonreplicating immunogen specifically excludes living vaccines such as attenuated viruses which are capable of reproducing themselves in a subject and continually presenting the subject with an immunologic stimulus. Instead, the term nonreplicating immunogen only encompasses vaccines such as killed viruses, peptides, proteins, including protein sub-unit immunogens such as those produced by genetic engineering techniques, and peptides bound to carriers, all of which are incapable of reproducing themselves in a subject. For most common avian diseases, the known vaccines which are nonreplicating immunogens, see, e.g., Murray, T.K., supra at 565, intended for post-hatch administration would be used in accordance with the inventive method, adjusting the dosage as necessary.

Because the present invention involves only the use of nonreplicating immunogens, there is, advantageously, no problem with the vaccine itself inducing lesions in the embryo and/or extraembryonic membranes, as discussed in Sharma, supra. Nevertheless, a consideration relating to the time frame for inoculation is the receptiveness of the inner egg structure to efficacious inoculation. The site of injection is preferably within either the region defined by the amnion, to include the amniotic fluid and the embryo itself, or else in the yolk sac. By the beginning of the fourth quarter of incubation, the amnion is sufficiently enlarged that penetration thereof is assured nearly all of the time when the injection is made from the center of the large end of the egg along the longitudinal axis. With a chicken egg in its eighteenth day of incubation, injection midway along, and perpendicular to, the longitudinal axis results in an amnion penetration frequency of about 80%, versus about 20% for the yolk sac. In the final quarter, the embryo is sufficiently developed and differentiated that it can tolerate the inherent randomization in the actual site of injection with no significant adverse effect on the rate of hatchability or on vital functions. Moreover, at this stage of incubation, the embryo is consistently positioned in the egg such that entry from the center of the large end will predictably result in injection in the upper dorsal region of the prenatal chick. The amniotic region in general, and the amniotic fluid in particular, is the preferred site of injection, because the yolk may carry maternal antibodies which would partially neutralize non-cell associated vaccines.

In addition, because younger embryos are more susceptible to infection than older embryos, care should be taken to reduce the chance of infection where younger embryos are injected. Appropriate steps include sterilization of injection needles, as explained below, and other procedures known in the art.

The mechanism of injection is not critical, but it is preferred that the method not unduly damage the tissues and organs of the embryo or the extraembryonic membranes surrounding it so that the treatment will not decrease hatch rate. A hypodermic syringe fitted with a needle of about #22 gauge is suitable for the purpose. A 1-inch needle, when fully inserted from the center of the large end of the egg, will penetrate the shell, the outer and inner shell membranes enclosing the air cell, and the amnion. Depending on the precise stage of development and position of the embryo, a needle of this length will terminate either in the fluid above the chick or in the chick itself. A pilot hole may be punched or drilled through the shell in order to prevent damaging or dulling of the needle.

It is envisioned that a high speed automated injection system for avian embryos will be particularly suitable for practicing the present invention. Numerous such devices are available, exemplary being the device disclosed in U.S. Patent Application Serial No. 881,121, filed on July 2, 1986 by John H. Hebrank. In this device, suction means simultaneously engage and lift a plurality of individual eggs from their respective upwardly facing portions and injects the eggs with the vaccine while the eggs are engaged by suction. At the same time, the device automatically transfers eggs from an incubation setting tray to a hatching tray. Most importantly, the device precisely positions the delivery ends of its injection needles at consistent locations within each individual egg regardless of the size of the eggs.

The following examples are provided to more fully illustrate the present invention:

## EXAMPLE 1

The efficacy of the present invention was demonstrated in a detailed study with over 300 birds, in which two different nonreplicating avian coccidiosis immunogens were tested over a range of concentrations for their ability to protect chicks against avian coccidiosis when injected into fertile eggs. The immunogens used were a sporulated E. tenella oocyst extract and a genetically engineered E. tenella antigen.

The E. tenella extract was obtained as follows: Sporulated E. tenella oocysts were stripped of their walls by treating them in a 1:1 volume/volume solution of commercially available "Clorox" chlorine solution and water for 15 minutes. Oocysts were washed in PBS and then placed in microcentrifuge tubes with glass beads and agitated in a refrigerated shaker bath (all steps were conducted at 4°C) to disrupt the oocysts. Phenylmethylsulfonyl flouride in a 1mM concentration in phosphate buffered saline was added as a protease inhibitor. Agitation was continued until all oocysts were ruptured, as determined by periodic microscopic inspection. Note that all oocysts and sporocysts present must be ruptured. The suspension was then centrifuged at 1,000 rpm for 10 minutes to settle debris, and the supernatant decanted and frozen for later use as the antigen. Antigen dose was calculated based upon protein determination using a modified Lowry method.

The procedure for obtaining the engineered antigen is described in Anderson and Mccandliss, Cloned Gene and Method for Making and Using the Same, a published international application by the Genex Corp. assigned International Publication No. WO 86/00528, and published on January 30, 1986. As explained therein, E. coli strain GX5408, deposited with the ATCC by that applicant and given accession No. 53154, is grown overnight at 37°C. in 10 ml of LB broth containing 100 ug/ml ampicillin. One liter of LB broth containing 100 ug/ml ampicillin in a two-liter flask is inoculated with the 10ml overnight culture and incubated with vigorous shaking at 37°C. When the $A_{600}$ of the culture reaches 0.6, 4 ml. of 0.1M IPTG is added and incubation is continued for two hours.

After two hours, the cells are harvested by centrifugation at 4°C. at 7,000 rpm for 10 minutes in a Sorvall GS-3 rotor. The cell pellet (about 2-3 g wet weight/L culture) is resuspended in 100 ml of 0.05M sodium phosphate, pH 7.0, and centrifuged again. The cells are resuspended in 0.05M sodium phosphate, pH 7.0 (5 ml/g wet weight of cells) and disrupted by sonication using a Branson Sonicator. Sonication is done for four 30-second bursts at full power with the cell suspension chilled in ice. The bursts are done at one-minute intervals. Cell debris is removed from the sonicated suspension by centrifugation at 4°C. at 15,000 rpm for 20 minutes in a Sorvall SM-24 rotor. The supernatant is removed and the gene product partially purified from it, as explained below.

The following procedures are done at 4°C. Nucleic acids are removed from the extract by slow addition of 0.1 volume of 30% streptomycin sulfate (30% w/v in water) followed by centrifugation for 10 minutes at 10,000 rpm in a Sorvall SS-34 rotor. To the supernatant solution, crystalline ammonium sulfate is added slowly with stirring to a final concentration of 36% (0.21 g $(NH_4)_2SO_4$/ml) The precipitated protein mixture containing the beta-galactosidase/coccidia antigen fusion protein is collected by centrifugation at 10,000 rpm for 10 minutes in a Sorvall SS-34 rotor. The protein pellet is dissolved in 0.05 M Tris•HCl, pH 7.5.

The protein solution is applied to a column (1.5 × 50 cm) of Sephacryl S-300 (Pharmacia) equilibrated in 0.05M Tris•HCl, pH 7.5. The protein is eluted with the same buffer. Column fractions are monitored for the presence of a beta-galactosidase/coccidia antigen fusion protein by SDS-polyacrylamide gel electrophoresis. Fractions containing the fusion protein are pooled and the proteins precipitated by adding ammonium sulfate to 36% as above. The protein is collected, dissolved in a minimum volume of .1 M sodium phosphate, pH 7.5 + 0.2mM dithiothreitol, and dialyzed extensively against the same buffer. SDS-polyacylamide gel electrophoresis will demonstrate that the fusion protein has a molecular weight of 140,000-160,000 daltons of which 115,000 daltons is beta-galactosidase and the remainder is coccidia antigen. The typical yield of fusion protein from one liter of culture is 10-20 mg of protein which contains

4

10-20% beta-galactosidase coccidia antigen fusion protein.

Fertilized broiler eggs were purchased from Carolina Golden Hatchery in Siler City, North Carolina and set in Jamesway 252 incubators. On day 17 of incubation the eggs were treated as follows: eggs in group 1 were injected with 10 microgram/150 microliter egg of E. tenella extract; eggs in group 2 were injected with 1 microgram/150 microliter egg of E. tenella extract; eggs in group 3 were injected with .1 microgram/150 microliter egg of E. tenella extract; eggs in group 4 were injected with .24 micrograms/150 microliters egg of engineered E. tenella protein; eggs in group 5 were injected with .12 micrograms/150 microliters egg of engineered E. tenella protein; eggs in group 6 were injected with .06 micrograms/150 microliters egg of engineered E. tenella protein; eggs in group 7 (a control group) were injected with 150 microliters egg of saline; eggs in group 8 (a control group) were not injected; and eggs in group 9 were injected with 2.8 micrograms/150 microliters egg of Lambda GT protein. A hatchery analysis of these treatments is presented in Table 1. These data indicate that saline injections

## TABLE 1

### HATCHERY ANALYSIS

| Group | # Eggs Treated | # Chicks Hatched | Dead in Hatcher | Early Dead | Middle Dead | Late Dead | Pipped | Hatch[1] of Fertile (%) |
|---|---|---|---|---|---|---|---|---|
| 1 | 46 | 42 | 0 | 0 | 0 | 2 | 2 | 91.3 |
| 2 | 45 | 40 | 0 | 3 | 0 | 2 | 0 | 88.9 |
| 3 | 45 | 26 | 0 | 4 | 1 | 13 | 1 | 57.8 |
| 4 | 45 | 22 | 5 | 4 | 1 | 13 | 0 | 60.0 |
| 5 | 44 | 26 | 1 | 2 | 1 | 7 | 7 | 61.4 |
| 6 | 45 | 25 | 2 | 2 | 0 | 10 | 6 | 60.0 |
| 7 | 46 | 41 | 0 | 3 | 0 | 2 | 0 | 89.1 |
| 8 | 45 | 37 | 0 | 2 | 0 | 4 | 2 | 82.2 |
| 9 | 42 | 35 | 0 | 1 | 1 | 4 | 1 | 83.3 |

$$[1]\text{Hatch of Fertile (\%)} = \frac{\text{Hatched + Dead in Hatcher}}{\text{\# Eggs Treated}} \times 100$$

(group 7) increased hatchability as compared to uninjected eggs (group 8). The two groups receiving high levels of E. tenella extract (groups 1 and 2) produced acceptable levels of hatchability as compared to saline inoculated and uninoculated eggs. Groups receiving a low level of E. tenella extract (group 3) and all three levels of engineered E. tenella immunogen (groups 4-6) showed reduced hatchability. Toxic effects were seen in the unhatched eggs.

Body weights were taken at one, two and four weeks of age (see Table 2). At one week of age, 10 chicks from each group were challenged with 20,000 live coccidial occysts/chick. Necropsies were performed one week after challenge. At three weeks of age an additional 10 chicks from each group were similarly challenged, and similarly necropsied one week later. Body weight (Table 2) and lesion scores in the intestine (Table 3) were used to assess severity of infection. Control groups 7 and 8 were combined for the compilation of these data.

## TABLE 2

### BODY WEIGHTS (gm)

| Group | 1 week Unchallenged | 2 Week Unchallenged | 2 Week Challenged | % Diff | 4 Week Unchallenged | 4 Week Challenged | % Diff |
|---|---|---|---|---|---|---|---|
| 1 | 159 | 350 | 293 | −16.3 | 1019 | 953 | −6.5 |
| 2 | 167 | 354 | 301 | −15.0 | 1007 | 949 | −5.8 |
| 3 | 172 | 360 | 319 | −11.4 | 997 | 1041 | +4.4 |
| 4 | 164 | 354 | 297 | −16.1 | — | 1049 | — |
| 5 | 156 | 344 | 291 | −15.4 | 1017 | 1001 | −1.6 |
| 7–8 | 166 | 354 | 293 | −17.2 | 1035 | 984 | −4.9 |
| 9 | 168 | 359 | 317 | −11.7 | 961 | 1048 | +9.1 |

## TABLE 3

### LESION SCORES[1]

| Group | 2 Week | 4 Week |
|---|---|---|
| 1 | 1.1 | 0.9 |
| 2 | 1.2 | 1.7 |
| 3 | 0.8 | 0.9 |
| 4 | 1.4 | 1.6 |
| 5 | 0.9 | 1.1 |
| 6 | 1.2 | 1.3 |
| 7–8 | 1.2 | 1.5 |
| 9 | 1.1 | 1.1 |

[1]Score assigned to each chick at necropsy based upon a scale of 0-4, with increasing severity indicated by progressively higher numbers.

While lesion scores were low in all groups, treatments 1, 3, 5, 6 and 9 had lower scores than the controls. Treatment groups 3 and 9 appeared to offer more limited protection against infection with E. tenella. Combined, these results show that the embryonic administration of nonreplicating immunogens is effective in increasing the resistance of chicks to avian coccidiosis.

EXAMPLE 2

ELISA tests were run on serum samples collected from 45 birds from the experiment described in example 1 above. Samples were collected prior to challenge infection, and frozen until use. The ELISA was run using serial dilutions of sera with E. tenella oocyst extract as the antigen in the wells. The ELISA procedure was conducted in accordance with the procedure described in Lillehoj, H.S. et al., Research in Avian Coccidiosis, 470, 471 (McDougald, L.R., Joyner, L.P., and Long, P.L., eds. 1986) (Published by the University of Georgia Department of Poultry Science). In this procedure, the optimum concentrations of antigens, antisera and substrate used were predetermined. Coccidial antigen was diluted in sodium carbonate buffer (pH 9.5). Fifty microliters of the antigen solution was dispensed into wells of flat-bottom immunoplates (Thomas Scientific, Philadelphia, Pennsylvania). The plates were incubated overnight at 4°C. The plates were then washed three times with PBS containing 0.05% Tween 20. The diluent used in this assay was PBS containing 1% BSA. PBS containing 0.05% Tween 20 was used as the washing buffer. Antigen-coated plates were treated with 10% BSA for 2 hours at room temperature to block non-specific binding sites. Antiserum was serially diluted 2 fold with a starting dilution of 1:16. After 2 hours of incubation at room temperature, the plates were washed four times with the washing buffer. Fifty microliters of rabbit or goat anti-chicken sera (Miles, Elkhart, Indiana) specific for heavy chains of chicken IgG, IgM and IgA were then added and plates were incubated for 1 hour at 37°C. The plates were washed four times and then incubated with 50 ul of biotin anti-rabbit or goat (Sigma, St. Louis, Missouri). After 30 minutes of incubation at 37°C, the plates were washed and incubated with 50 ul of streptavidin-peroxidase (Zymed, San Francisco, California). Enzyme reaction was initiated by the addition of ortho-phenylenediamine dihydrochloride (OPD) dissolved in 0.05M citrate phosphate buffer containing fresh $H_2O_2$. Optical density was read at 450 nm with a multichannel spectrophotometer (Titertek Multiskan, Flow Laboratories).

The results of these procedures are set forth in Table 4. The ELISA end point given represents the average of 3 pools of 3 serum samples.

## TABLE 4

### ELISA RESULTS

| Antigen | Level/Embryo in Micrograms | ELISA End Point |
|---|---|---|
| Sporulated E. tenella Oocyst Extract | .1 | 1:2987 |
| | 1.0 | 1:1280 |
| | 10.0 | 1:1920 |
| Engineered E. tenella Protein | .24 | 1:640 |
| Saline | | 1:427 |

These results confirm that a substantial immune response was induced by the treatment of the present invention, as best shown by comparison of the ELISA end point for the saline control group with the ELISA end point for the group administered .1 micrograms (the smallest dose) of sporulated E. tenella oocyst extract.

The foregoing examples have been provided to illustrate the present invention. While specific terms are employed, they are used in a generic, descriptive sense only and not for purposes of limitation, the scope of the invention being defined by the following claims. Equivalents of the claims are to be included therein.

## Claims

1. A method for treating a fertilized bird's egg to control an immunizable disease which comprises injecting into the egg a non-replicating immunogen effective for inducing immunity against the disease in the bird, while the bird is an embryo enclosed within the egg.

2. A method according to claim 1, wherein said nonreplicating immunogen is administered during about the final quarter of the incubation period of the bird within the egg.

3. A method according to claim 1 or claim 2, wherein the egg is a chicken, turkey, duck, goose, quail or pheasant egg.

4. A method according to any preceding claim, wherein the immunogen is injected into the egg within the region defined by either the amnion or the yolk sac.

5. A method according to claim 4, wherein the immunogen is injected into the amniotic fluid.

6. A method according to any preceding claim, wherein the immunogen is a coccidiosis immunogen.

7. A method according to any preceding claim, wherein the immunogen is an Eimeria extract.

8. A method according to claim 7 wherein the immunogen is a sporulated *Eimeria* *tenella* oocyst extract.

9. A fertile bird egg characterized by having deposited therein a foreign immunogen effective for inducing immunity to an immunizable disease in the hatchling of said egg, wherein said immunogen is a nonreplicating immunogen.

10. A bird egg according to claim 9. which is a chicken, turkey, duck, goose, quail, or pheasant egg.

11. A bird egg according to claim 10, which is a chicken egg.

12. A bird egg according to any one of claims 9 to 11 wherein said immunogen is an *Eimeria* extract.

K. S. WARREN
F. C. WARREN
A. R. WARREN
D. A. KELTIE
R. O. GRAY

S. C. D. BANKES
S. E. BRIGHT

TRADE MARKS
P. D. ADCOCK
D. M. ARMSTRONG

CONSULTANT
C. G. B. HOSE

## BARON & WARREN

CHARTERED PATENT AGENTS
EUROPEAN PATENT ATTORNEYS
18 SOUTH END
KENSINGTON
LONDON W8 5BU

PATENTS TRADE MARKS
DESIGNS COPYRIGHT

TELEPHONE
01-937 0294-5
01-937 4104-5

TELEX
21319-PATAGS

TELECOPIER
01-937 4786

CABLES
PATAGS-LONDON W8

26 April 1988

OUR REF SCDB/CF.

European Patent Office
P.B 5818 PATENTLAAN 2
2280 HV RIJSWIJK (ZH)
THE NETHERLANDS.

YOUR REF

. Dear Sirs

Re:   EUROPEAN PATENT APPLICATION NO. 88303349.0.
      EMBREX INC. AND ANOTHER.

It has come to our attention that the United States of America should not be a co-applicant in this case. One of the three Inventors, Michael D. Ruff, executed an assignment of his share in the invention to the U.S. Department of Agriculture but it now transpires that this assignment was for the U.S.A. only. Mr. Ruff thus retained for himself the right to file a European application.

We therefore respectfully request leave to correct the application form under Rule 88 EPC, by substitution of the attached additional sheet for the additional sheet originally attached to the application form.

Also attached is the required Designation of Inventor, with an extra copy for each of the Inventors who is not an applicant.

We should perhaps mention that the assignment referred to in the designation of inventor was an assignment from the Inventors Smith and Thaxton to Embrex Inc. Embrex Inc. thus become co-applicants with the third Inventor Mr. Ruff.

If there are any difficulties regarding the amendment of the application form we would suggest contacting us by telephone.

Yours faithfully